# EUROPEAN PATENT APPLICATION

(11) **EP 3 315 955 A1**
(43) Date of publication of application: **02.05.2018**
(21) Application number: 17198383.6
(22) Date of filing: 25.10.2017
(51) Int. Cl.: G01N 27/04, A61F 13/49, A61B 5/00, G01N 27/07, A61F 5/48

(54) **INCONTINENCE DETECTION PAD WITH USER ADJUSTABLE SENSITIVITY**

(30) Priority: 27.10.2016 US 201662413630 P
(71) Applicant: Hill-Rom Services, Inc., Batesville, IN 47006-9167 (US)
(72) Inventor: WILLIAMS, Joshua A, Batesville, IN Indiana 47006-9167 (US); WIGGERMANN, Neal, Batesville, IN Indiana 47006-9167 (US); FU, Yongji, Batesville, IN Indiana 47006-9167 (US); SRIVASTAVA, Varad, Batesville, IN Indiana 47006-9167 (US)
(74) Representative: Findlay, Alice Rosemary

(57) **Abstract**

A disposable incontinence detection system (100) monitors an area for incontinence events. The incontinence detection system includes a substrate (110) having a length and a width defining a monitoring area. Additionally, the incontinence detection system includes a moisture sensor (120) positioned within the monitoring area. The moisture sensor includes a circuit that includes traces (132, 134, 136) spaced apart from each other such that the presence of moisture bridging a space between at least two of the traces will close the circuit. Additionally, the circuit includes a removable conductor (150) coupled to one of the traces such that removal of the conductor irreversibly prevents the trace from closing the circuit. The moisture sensor is configured to determine whether the circuit has been closed.

## Description

The present disclosure relates to incontinence detection systems and particularly, to incontinence detection systems that detect changes in electrical properties of a pad due to moisture. More particularly, the present disclosure relates to incontinence detection systems in which the sensitivity of the system is adjustable.

In general, prolonged exposure of a person's skin to biofluids such as urine may cause breakdown of the skin and potentially lead to ulcers. Accordingly, it is advantageous to detect and remedy such exposure as soon as possible. Some systems exist for detecting the presence of biofluids in a substrate, such as a layer on a hospital bed or a diaper. However, due to variations in the amount that different people perspire, some of these systems may generate false positives by detecting sweat instead of urine. As a result, caregivers may unnecessarily spend time inspecting and/or changing hospital bed sheets or diapers that may not actually contain harmful levels of biofluids.

The present application discloses one or more of the following features alone or in any combination.

According to one aspect of the present disclosure, a disposable incontinence detection system for monitoring an area for incontinence events is provided. The incontinence detection system includes a substrate having a length and a width that define a monitoring area. A moisture sensor is positioned within the monitoring area and includes a circuit that includes a plurality of traces that are spaced apart from each other such that the presence of moisture bridging a space between at least two of the traces will close the circuit. Additionally, the moisture sensor includes a removable conductor coupled to one of the plurality of traces such that removal of the conductor irreversibly prevents the trace from closing the circuit. The moisture sensor is configured to determine whether the circuit has been closed (e.g., as a result of an incontinence event).

In some embodiments, the distances between each trace of the plurality of electrically conductive traces may correlate to predetermined volumes of moisture. Alternatively or additionally, the distances between each trace of the plurality of electrically conductive traces may be predetermined based on the area of the substrate and the absorption properties of the substrate. The plurality of electrically conductive traces includes may include at least three electrically conductive traces, for example. In some embodiments, the at least three electrically conductive traces may be positioned in predetermined parallel distances from each other in order to detect 50mL and 100mL moisture volumes, and/or other moisture volumes.

Further, according to the present disclosure, a method for modifying the sensitivity of a disposable incontinence detection system is provided. The disposable incontinence detection includes a substrate having a length and a width defining a monitoring area, and a moisture sensor that is positioned within the monitoring area and includes a circuit having a plurality of traces spaced apart from each other such that the presence of moisture bridging a space between at least two of the traces will close the circuit, and a removable conductor coupled to one of the plurality of traces such that removal of the conductor irreversibly prevents the trace from closing the circuit. The moisture sensor is configured to determine whether the circuit has been closed. The method includes irreversibly removing the conductor from the trace.

The invention will now be further described by way of example with reference to the accompanying drawings, in which:
Fig. 1 is a schematic view of an embodiment of a disposable incontinence detection system including a removable conductor to adjust a sensitivity of the incontinence detection system;
Fig. 2 is a schematic view of an embodiment of the disposable incontinence detection system of Fig. 1 with the removable conductor removed;
Fig. 3 is a block diagram of at least one embodiment of a moisture sensor included in the disposable incontinence detection system of Figs. 1 and 2;
Fig. 4 is a schematic view of an alternative embodiment of the disposable incontinence detection system that includes multiple removable conductors to adjust the sensitivity of the incontinence detection system;
Fig. 5 is a schematic view of the incontinence detection system of Figs. 1 2, and 4, positioned underneath a patient on a patient support apparatus;
Fig. 6 is a schematic view of an alternative embodiment of the disposable incontinence detection system incorporated into a diaper;
Figs. 7A-D are exploded views of layers of components that may be combined in alternative embodiments of the disposable incontinence detection system;
Fig. 8 is a simplified flow diagram of a method for preparing the disposable incontinence detection system for operation; and
Fig. 9 is a simplified flow diagram of a method that may be performed by embodiments of the disposable incontinence detection system to detect an incontinence event.

The present disclosure relates to systems and methods for detecting incontinence-caused events associated with a person being monitored. Thus, it should be appreciated that the systems described herein are able to detect biofluids such as blood, urine, fecal matter, interstitial fluid, saline, or any other fluid having a large concentration of ions that easily conduct electricity. Urine, for example is relatively conductive (in the range of 0.06 to 0.1 siemens per meter (S/m)). Blood is much more conductive (in the range of 1.18 to 3.35 S/m). Fecal conductivity is typically much lower, but more variable depending upon ion content. Values of 0.06 S/m are typical. The term "incontinence" as used herein is intended to cover all of these biofluids. The present disclosure further describes systems and methods for adjusting the sensitivity of such incontinence detection systems to avoid false positives (i.e., a determination that an incontinence event such as a bowel movement or urination occurred when it did not) caused by perspiration. By reducing the number of false positives, caregivers such as nurses may reduce the amount of time spent investigating whether detections of incontinence events are erroneous and focus more of their time on removing soiled sheets, garments, and other materials from patients who have actually experienced an incontinence event.

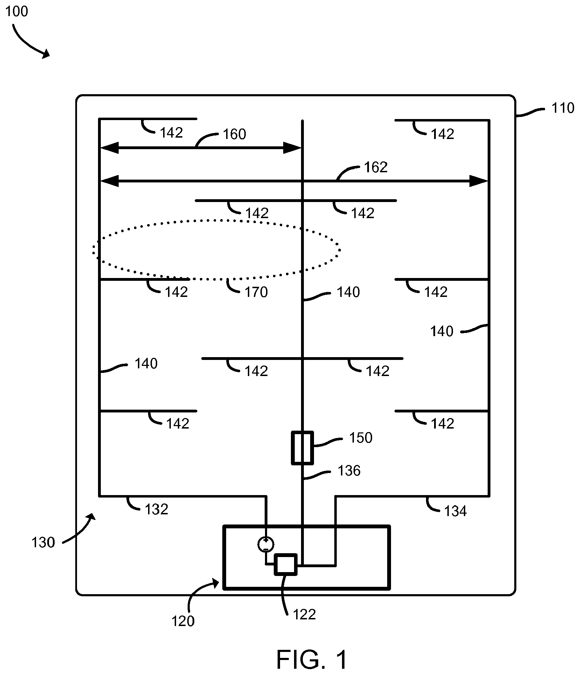

Referring now to Fig. 1, a disposable incontinence detection system 100 to detect the presence of a selected volume of moisture as an incontinence event is shown. The disposable incontinence detection system 100 includes a substrate 110 that may rest upon or be integrated into a patient support apparatus, such as a hospital bed, chair, wheelchair, or stretcher. Alternatively, the disposable incontinence detection system 100 may be incorporated into a garment or diaper to be worn by a patient, as described in more detail herein. The illustrative disposable incontinence detection system 100 additionally includes a moisture sensor 120 that includes a plurality of electrically conductive traces 130. The traces 130 include a trace 132 and another trace 134 positioned opposite the trace 132 by a distance 162. The traces 130 also include another trace 136 positioned about halfway between the traces 132 and 134. In other words, the trace 136 is positioned a second distance 160 away from the trace 132. Each trace 130 includes longitudinal segments 140 that extend along a length of the substrate 110 and lateral segments 142 that extend laterally from the longitudinal segments 140. As shown in Fig. 1, the lateral segments 142 are interdigitated to enhance the ability of the moisture sensor 120 to detect areas of moisture that may be oriented parallel to the longitudinal segments 140. In use, the moisture sensor 120 applies a voltage to the trace 132. If a volume of biofluid 170 spans the distance 160, an electrical current passes through the biofluid from the trace 132 to the trace 136, and is detected by the moisture sensor 120. Importantly, the current passes through a removable conductor 150, which is initially (i.e., at the time of manufacture) coupled to the trace 136. The removable conductor 150 is any device that is capable of conducting an electrical current and being permanently removed from the trace 130 to prevent current from passing through the trace 130. In the illustrative embodiment, the removable conductor is an electrically conductive sticker. In response to detecting the electrical current (e.g., a predefined change in the voltage difference between the traces 130), the moisture sensor 120 is configured to activate a notification device 122 to alert a caregiver of the occurrence of an incontinence event. The notification device 122 may include a visual output device, an audio output device, and/or a data communication subsystem to produce a notification of an occurrence of an incontinence event in one or more formats, as described in more detail herein.

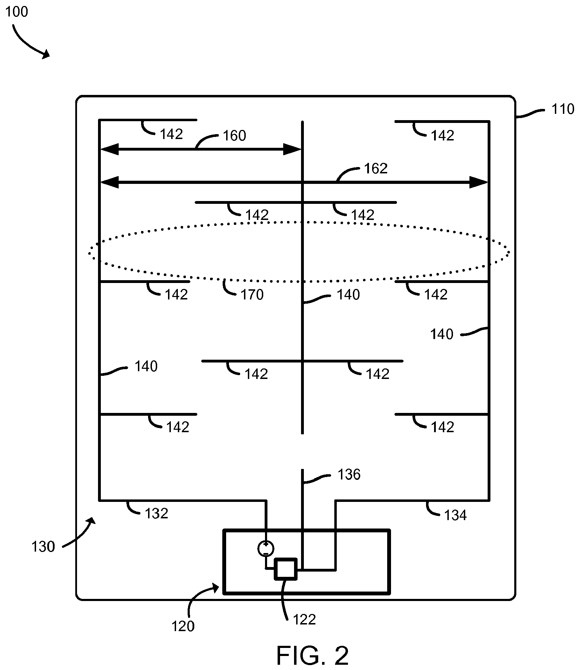

Referring now to Fig. 2, the removable conductor 150 may be permanently decoupled from the trace 136 to prevent an electrical current from flowing through the trace 136 and completing a circuit between the traces 132, 136. Rather, a larger volume of the biofluid 170 must span the distance 162 between the traces 132, 134 in order for the moisture sensor to detect the current (e.g., a predefined change in the voltage difference between the traces 130). Accordingly, by removing the removable conductor 150 from the disposable incontinence detection system 100, a person may change the sensitivity of the system 100. This is advantageous because a patient may normally produce a certain amount of sweat or other biofluid that is not related to an incontinence event but which may otherwise complete the circuit between the traces 132 and 136. In such circumstances, by removing the removable conductor 150, the patient must release a larger volume of biofluid (enough to electically connect the traces 132 and 134) in order for the moisture sensor 120 to activate the notification device 122. Other patients who may not produce as much sweat or other biofluid may benefit from a more sensitive configuration of the disposable incontinence detection system 100 in which the removable conductor 150 is not removed.

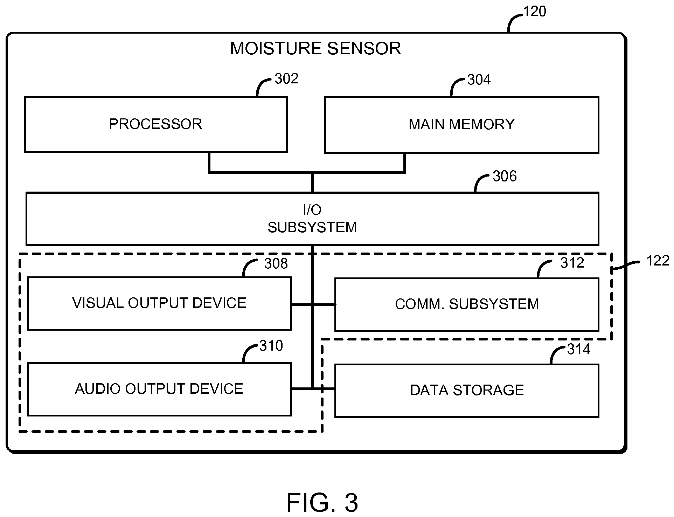

Referring now to Fig. 3, the moisture sensor 120 may be embodied as any type of electronic device, such as a computer or microcontroller, capable of performing the functions described herein. As shown in Fig. 3, the illustrative moisture sensor 120 includes a processor 302, a main memory 304, an input/output subsystem 306, one or more of a visual output device 308, an audio output device 310, and a data communication subsystem 312 as components of the notification device 122, as well as a data storage device 314. Of course, the moisture sensor 120 may include other or additional components in other embodiments. Additionally, in some embodiments, one or more of the illustrative components may be incorporated in, or otherwise form a portion of, another component. For example, the memory 304, or portions thereof, may be incorporated in the processor 302 in some embodiments.

The processor 302 may be embodied as any type of processor capable of performing the functions described herein. For example, the processor may be embodied as a microcontroller, single or multi-core processor(s) having one or more processor cores, or other processor or processing/controlling circuit. Similarly, the main memory 304 may be embodied as any type of volatile or non-volatile memory or data storage capable of performing the functions described herein. In operation, the main memory 304 may store various data and software used during operation of the moisture sensor 120 such as threshold voltage levels indicative of a closed circuit between one or more of the traces 130, and in some embodiments, the type of biofluid closing the circuit, given that different biofluids have different degrees of electrical conductivity, as well as network communication settings, libraries, drivers, programs, applications, and operating systems. The main memory 304 is communicatively coupled to the processor 302 via the I/O subsystem 306. Of course, in other embodiments (e.g., those in which the processor 302 includes a memory controller), the main memory 304 may be directly communicatively coupled to the processor 302.

The I/O subsystem 306 may be embodied as circuitry and/or components to facilitate input/output operations with the processor 302, the main memory 304, and other components of the moisture sensor 120. For example, the I/O subsystem 306 may be embodied as, or otherwise include, memory controller hubs, input/output control hubs, firmware devices, communication links (i.e., point-to-point links, bus links, wires, cables, light guides, printed circuit board traces, etc.) and/or other components and subsystems to facilitate the input/output operations. In some embodiments, the I/O subsystem 306 may form a portion of a system-on-a-chip (SoC) and be incorporated, along with the processor 302, the memory 304, and other components of the moisture sensor 120, on a single integrated circuit chip.

As described above, the illustrative moisture sensor 120 includes the notification device 122, which may include one or more of a visual output device 308, an audio output device 310, or a data communication subsystem 312. Of course, in other embodiments, the notification device 122 may include additional or other output devices. The visual output device 308 may be embodied as any type of device capable of providing visual signal to an observer of the notification device 122. The visual signals may be lights or graphical indications of the occurrence of an incontinence event. Accordingly, the visual output device 308 may be embodied as, or otherwise use, any suitable display technology including, for example, a light emitting device, such as a light emitting diode (LED), or a graphical display such a liquid crystal display (LCD), a light emitting diode (LED) display, and/or other display usable in a compute device.

The audio output device 310 may be embodied as any type of device capable of providing an audible signal indicative of an occurrence of an incontinence event, such as a speaker. The audible signal may be a constant tone, a repeating audible pattern, speech, or other sound. The data communication subsystem 312 may be embodied as one or more devices and/or circuitry capable of enabling communications with one or more other electronic devices over a network. The communication subsystem 312 may be configured to use any suitable communication protocol to communicate with other devices including, for example, wireless data communication protocols, cellular communication protocols, and/or wired communication protocols.

The moisture sensor 120 may additionally include a data storage device 314, which may be embodied as any type of device or devices configured for short-term or long-term storage of data such as, for example, memory devices and circuits, memory cards, hard disk drives, solid-state drives, or other data storage devices. The data storage device 314 may store data and software used during operation of the moisture sensor 120 such as threshold voltage levels indicative of a closed circuit between one or more of the traces 130, and in some embodiments, the type of biofluid closing the circuit, given that different biofluids have different degrees of electrical conductivity, as well as network communication settings, libraries, drivers, programs, applications, and operating systems.

In some embodiments, the moisture sensor 120 does not include one or more of the processor 302, main memory 304, I/O subsystem 306 or data storage device 314, and the notification device 122 is activated directly by the electrical current passing through the circuit closed by the biofluid 170.

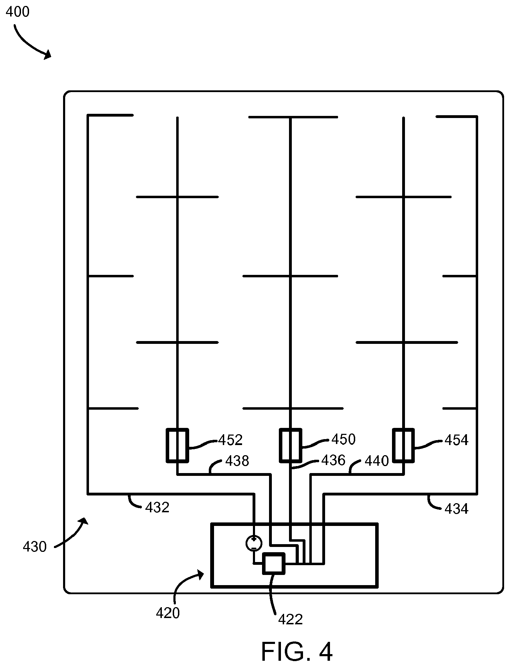

Referring now to Fig. 4, an alternative embodiment of a disposable incontinence detection system 400 includes five traces 430, including a trace 432, another trace 434 positioned opposite the trace 432, as well as a trace 436 having a removable conductor 450 coupled thereto, and traces 438 and 440 positioned on either side of the trace 436, each having a respective removable conductor 452, 454 coupled thereto. Additionally, the disposable incontinence detection system 400 includes a moisture sensor 420 that includes a notification device 422. The moisture sensor 420 and the notification device 422 are similar to the moisture sensor 120 and notification device 122 described above, except the moisture sensor 420 is configured to activate the notification device 422 in response to electrical current passing through the trace 432 and any one or more of the traces 434, 436, 438, or 440. By including more traces with removable conductors, compared to the disposable incontinence detection system 100 of Figs. 1 and 2, the disposable incontinence detection system 400 provides finer control over the sensitivity of the moisture sensor. More specifically, a caregiver may choose to keep all of the removable conductors 450, 452, 454 coupled to their respective traces 436, 438, 440 to provide a higher level of sensitivity than the configuration of the incontinence detection system of Fig. 1 would provide, given that additional traces (e.g., trace 438) would be coupled to the moisture sensor 420 and able to conduct an electrical current to the moisture sensor 420 if a smaller volume of biofluid was present (e.g., a volume of biofluid that spanned only the distance between the traces 432 and 438). Alternatively, the caregiver may remove one or more of the removable conductors 450, 452, 454 to provide other levels of sensitivity, including those provided by the disposable incontinence detection system 100. As should be understood from the foregoing, the sensitivity of the disposable incontinence detection systems 100, 400 is a function of the number of traces equipped with removable conductors. It is within the scope of this disclosure that other embodiments of the incontinence detection system may be equipped with other amounts of traces and removable conductors.

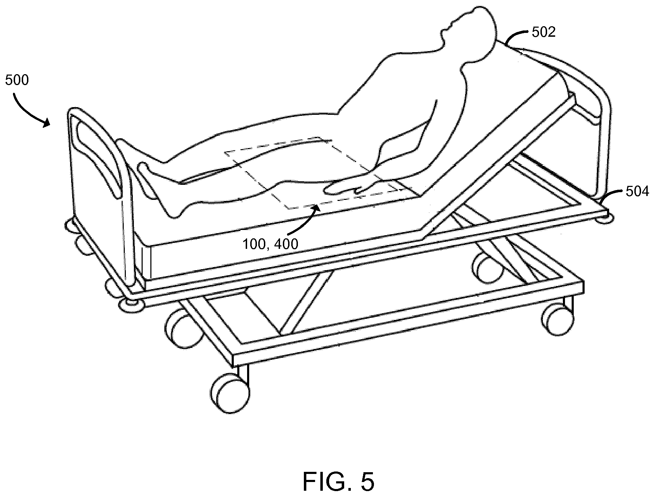

Referring now to Fig. 5, the disposable incontinence detection system 100, 400 may be placed underneath a patient positioned on a patient support apparatus 500. In the illustrative embodiment, the patient support apparatus 500 is a hospital bed having a frame 504 and a mattress 502. In other embodiments, the patient support apparatus may be any device capable of physically supporting all or a portion of the patient, such as a chair, a wheel chair, or a stretcher. The disposable incontinence detection system 100, 400 is illustratively placed atop the mattress 502, in a zone where biofluid from an incontinence event is to be received. Upon the occurrence of an incontinence event, the disposable incontinence detection system 100, 400 may be quickly removed from the mattress 502 in the process of changing the clothing and sheets of the patient. In other embodiments, the disposable incontinence detection system 100, 400 may instead by incorporated into the mattress 502.

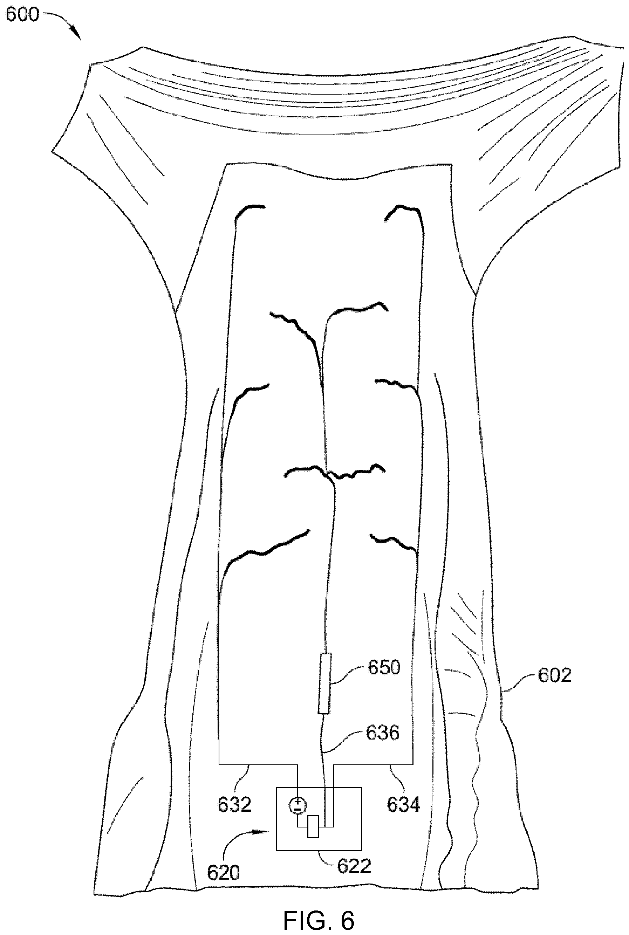

Referring now to FIG. 6, an embodiment of the disposable incontinence detection system 600 may be incorporated into a diaper 602. The disposable incontinence detection system 600 includes a moisture sensor 620 and a notification device 622 that are similar to the moisture sensor 120 and notification device 122 described above. Further, the disposable incontinence detection system 600 includes traces 632, 634, and 636 in a similar arrangement to that of the incontinence detection system 100. Further, a removable conductor 650 is coupled to the trace 636 in a similar manner to the removable conductor 150, described above. In the illustrative embodiment of the disposable incontinence detection system 600, the traces 632, 634, 636 are flexible to allow them to bend with the diaper 602 as the wearer moves and changes positions. Further, in embodiments in which the notification device 622 is configured to emit a visual signal, the visual output device 308 may be oriented outwards from the diaper 602 when it is worn, to enable the visual signal to be more readily noticed by a caregiver.

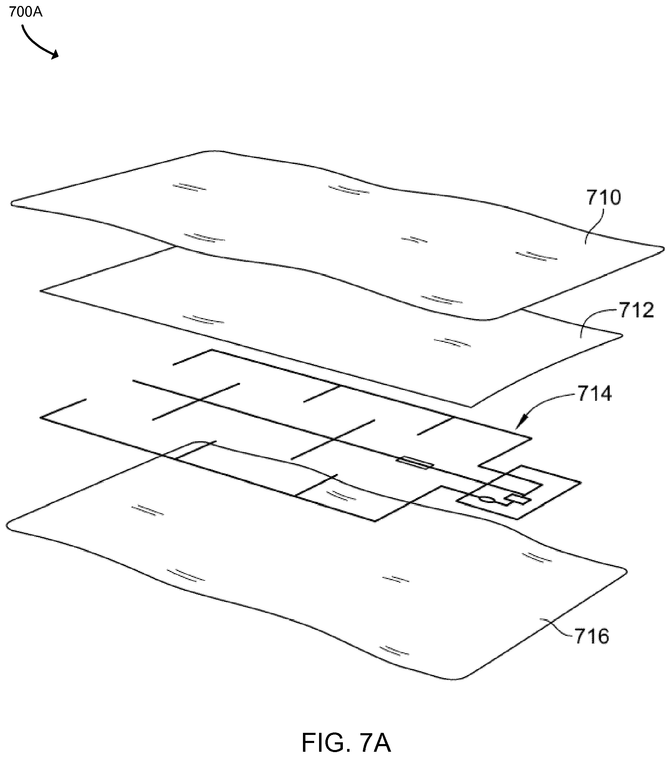

Referring now to Figs. 7A-7D, embodiments 700A, 700B, 700C, 700D of the disposable incontinence detection system may be formed as a pad of multiple layers of components. The embodiments shown in Figs. 7A-7D are configured as pads to support a patient on a top surface and are typically used with a patient support, frame, or bed. Referring now to a first embodiment 700A, shown in Fig. 7A, a top surface includes a non-woven layer 710, in contact with an absorbent layer 712, and traces 714 of the moisture sensor, similar to the traces 130 of the moisture sensor 120, are printed directly onto a barrier layer 716 which defines the bottom surface of the pad 700A. The non-woven top layer 710 typically is a polymer-based material and is made from bonded fibers and/or filaments. The non-woven top layer 710 provides comfort and softness for a patient on the pad 700A. The absorbent layer 712 provides a core material of the pad to hold moisture and may include, for example, wood pulp and/or extruded polymer fibers. The fibers can be loose or bonded into a web of material at the option of the pad designer.

The traces 714 of the moisture sensor include conductive ink traces. Suitable conductive inks include, for example, carbon, silver, copper, zinc and graphene. The barrier layer 716 is typically polyethylene (PE) which provides a barrier to prevent moisture penetration to a support surface or frame beneath the pad 700A. Polypropylene (PP) sheets and/or polyurethane (PU) sheets are also acceptable to be used as the barrier layer 716. The barrier layer 716 may or may not be breathable. In some embodiments, the barrier layer 716 is substantially waterproof. In an example embodiment, silver ink is printed on a sheet of PE material that is inserted into a pad that, itself, has a bottom substantially waterproof layer. The layer of PE material with silver ink is situated between the bottom layer and an overlying layer of absorbent material.

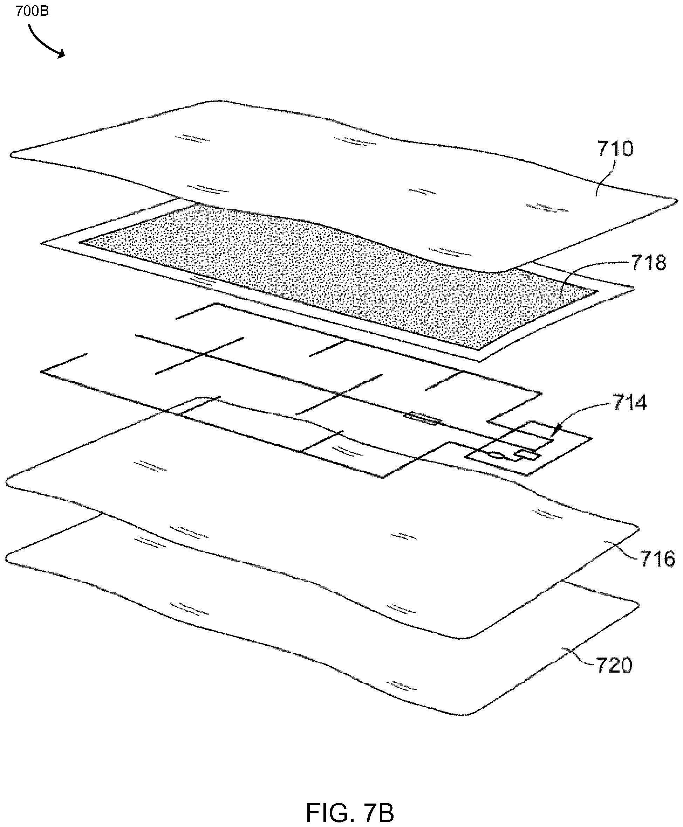

In another exemplary embodiment pad 700B, as shown in Fig. 7B, a top surface non-woven layer 710 is provided in contact with an absorbent layer 718 with a super absorbent polymer beneath it. Traces 714 are printed directly on the barrier layer 716 and a non-woven layer 720 is provided beneath the barrier layer 716 to contact a patient support or frame. In this embodiment, an absorbent layer with super absorbent polymer 718 is provided. The super absorbent polymer (SAP) provides 3-5 times more moisture absorption than the wood pulp absorbent layer 712 of pad 700A of Fig. 7A. The SAP will typically be incorporated into absorbent layer 718 as powder but can be fibers if desired. An example of a SAP that may be used in absorbent layer 718 is sodium polyacrylate. Alternatively or additionally, the SAP may be impregnated into layer 718 or be situated on top of layer 718 in other embodiments. The non-woven bottom layer 720 is optional and may be added to provide friction to prevent sliding with respect to a patient support or frame. The non-woven bottom layer 720 may also be added to provide grip when moving the pad 700B and to provide increased tensile strength for moving patients.

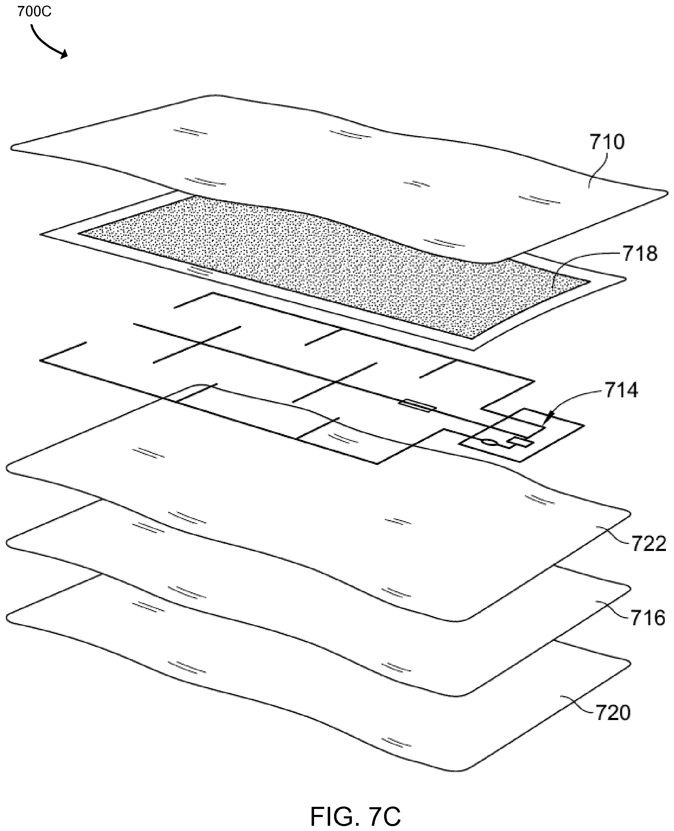

In another exemplary embodiment, as shown in Fig. 7C, a pad 700C includes a non-woven layer 710 at the top surface, an absorbent layer with SAP 718, the traces 714 printed on a top surface of a non-woven substrate 722 between the absorbent layer with SAP 718 and the bottom of the non-woven substrate 722, barrier layer 716 and non-woven layer 720 at the bottom. In this embodiment, non-woven substrate 722 of pad 700C provides an alternative to printing on the barrier sheet 716 if, for example, the barrier sheet 716 is made of a material that is not capable of surviving the print curing process required to print the conductive ink traces onto the barrier layer 716. The non-woven substrate 722 may also provide additional tensile strength, thereby preventing electrode and/or pad rupture and stretching. In a variant of pad 700C of Fig. 7C, the traces 714 are printed on a bottom surface of the non-woven substrate 722 that faces the barrier layer 716. This provides an extra layer that moisture from a patient on the top surface must wick through. This may enhance the reduction of false positives for incontinence detection caused by perspiration or other undesirable moisture detection, for example.

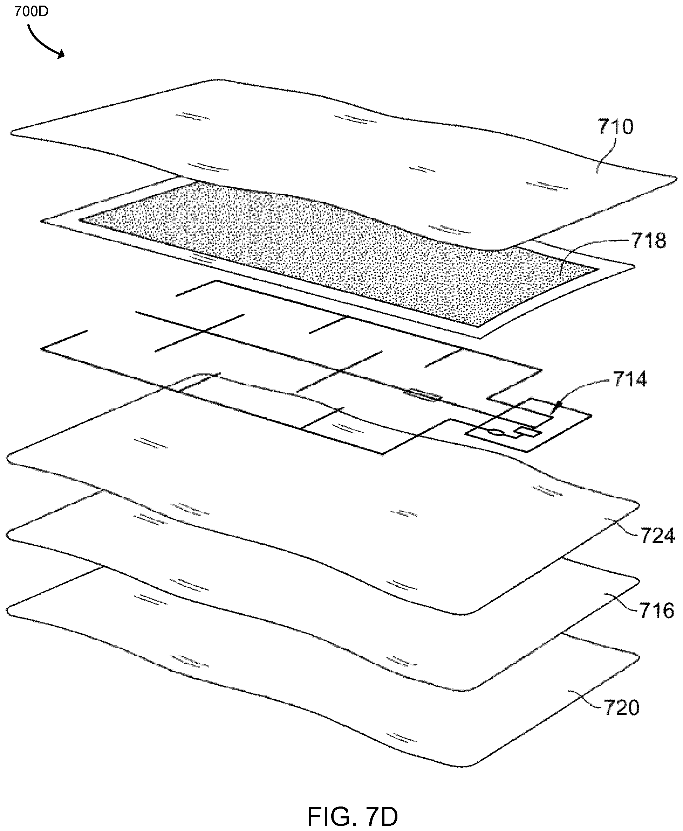

In another exemplary embodiment, as shown in Fig. 7D, pad 700D includes a non-woven top layer 710, an absorbent layer with SAP 718, traces 714 printed onto film substrate 724 which is a co-laminate with the barrier layer 716, and non-woven layer 720. Absorbent layer 712 and absorbent layer with SAP 718 may be alternatively used in any of the embodiments depending on the desired level of moisture retention. Similarly, non-woven layer 720 may be optionally provided or removed from any of the embodiments depending on the desirability of the features it provides.

In the illustrative examples of pads 700A-700D, each of layers 710, 712 716, 718, 720, 722, 724, to the extent present, are generally rectangular in shape. However, layers 712, 718 are slightly smaller in length and width dimensions than layers 710, 716, 720, 722, 724. The larger-sized layers 710, 716, 720, 722, 724 are attached at their peripheries (again, to the extent present in any particular embodiment 700A-D) such as by sewing, sonic or RF welding, adhesive or other laminating technique. Thus, the periphery of the absorbent material layers 712, 718 is inset by a small amount from the overall periphery of pads 700A-D. The traces 714 are within the periphery of the absorbent layers 712, 718 and so are also inset from the overall periphery of the pads 700A-D. In other embodiments, the layers 710, 712, 716, 718, 720, 722, 724 are attached to each other across their entire widths and not just at the peripheries.

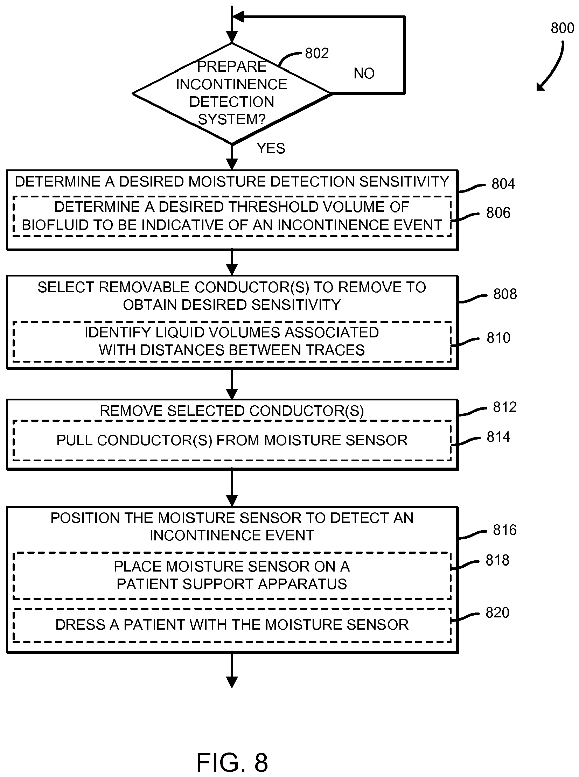

Referring now to Fig. 8, a caregiver may perform a method 800 for preparing the disposable incontinence detection system (e.g., the disposable incontinence detection system 100) for operation. The method 800 begins with block 802 in which the caregiver determines whether to prepare a disposable incontinence detection system 100 for use. In the illustrative embodiment, the caregiver may be in the process of changing the bedding of a patient, may be preparing a patient transport apparatus, such as a stretcher or wheel chair with the incontinence detection system, may be preparing a wearable embodiment of the disposable incontinence detection system (e.g., the wearable disposable incontinence detection system 600), or may be preparing another embodiment of the disposable incontinence detection system for use. Regardless, if the caregiver determines to proceed with preparing the disposable incontinence detection system for use, the method 800 advances to block 804. In block 804, the caregiver determines a desired moisture detection sensitivity for the disposable incontinence detection system. As indicated in block 806, in the illustrative embodiment, the caregiver determines a desired threshold volume of biofluid (e.g., 100 milliliters) that, if present, is indicative of an incontinence event for a particular patient. As described above, different patients may produce different amounts of sweat or other biofluids that are not associated with incontinence. Accordingly, for patients that produce relatively large volumes of such biofluids, a caregiver would reduce the sensitivity of the disposable incontinence detection system by setting the threshold volume of biofluid to a larger amount than would be desired for a patient for whom a smaller amount of biofluid would be indicative of an incontinence event (e.g., a patient who sweats less).

In block 808, the caregiver selects one or more removable conductors to remove from the disposable incontinence detection system to obtain the desired level of sensitivity. In doing so, as indicated in block 810, the caregiver may identify bioliquid volumes associated with distances between the traces 130. For example, if the caregiver is using the disposable incontinence detection system 100, the caregiver may determine whether to keep the removable conductor 150 in place, to detect the presence of a first volume (e.g., 50 milliliters) of biofluid or to remove the removable conductor 150 to be sensitive to no less than a second volume (e.g., 100 milliliters) of biofluid, which is greater than the first volume. If the caregiver is instead using another embodiment that includes multiple removable conductors, such as the removable conductors 450, 452, 454 of the disposable incontinence detection system 400, the caregiver may select one or more of the removable conductors 450, 452, 454. In some embodiments, the disposable incontinence detection system may include labels or other indicia of volumes of biofluid that may be detected with one or more of the removable conductors in place or removed, and/or other indications of levels of sensitivity (high, medium, low, etc.) associated with different configurations of the one or more removable conductors.

In block 812, the caregiver removes the one or more selected conductors from the disposable incontinence detection system. For example, if the caregiver is using the disposable incontinence detection system 100, the caregiver may remove the removable conductor 150. If the caregiver is instead using another embodiment that includes multiple removable conductors, such as the removable conductors 450, 452, 454 of the disposable incontinence detection system 400, the caregiver may remove one or more of the removable conductors 450, 452, 454. As indicated in block 814, in removing the selected one or more removable conductors, the caregiver may pull them away from the moisture sensor, thereby preventing the associated traces 130 from being able to conduct an electrical current to the notification device 122.

In block 816, the caregiver positions the moisture sensor 120 of the disposable incontinence detection system 100 to detect an incontinence event. In doing so, as indicated in block 818, the caregiver may place the moisture sensor on a patient support apparatus. In other embodiments, as indicated in block 820, the caregiver may dress the patient with the moisture sensor, such as when the disposable incontinence detection system is incorporated into a diaper (e.g., the disposable incontinence detection system 600) or other garment. In the illustrative embodiment, the caregiver positions the moisture sensor (e.g., the moisture sensor 120), and in particular, the traces (e.g., the traces 130), in a position to receive the majority of any biofluid released by the patient in an incontinence event, such as within a predefined distance of the genitals and/or buttocks of the patient.

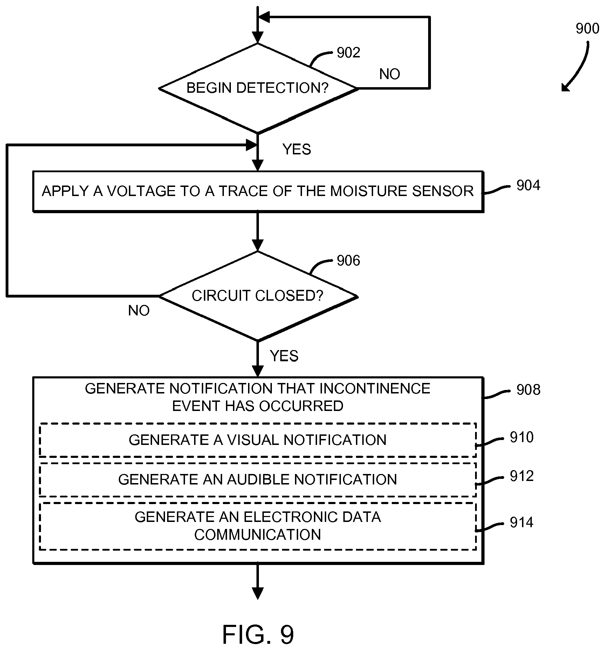

Referring now to Fig. 9, in use, the disposable incontinence detection system 100 may perform a method 900 for detecting an incontinence event. The method 900 begins with block 902, in which the incontinence detection system 100 determines whether to begin detection for an incontinence event. In the illustrative embodiment, the disposable incontinence detection system 100 is configured to begin detection upon receiving power. In other embodiments, the disposable incontinence detection system 100 may await an activation signal, such as through the data communication subsystem 312 from another compute device, or from another source. Regardless, if the disposable incontinence detection system 100 determines to begin detection, the method 900 advances to block 904 in which the disposable incontinence detection system 100 applies a voltage to a trace (e.g., the trace 132) of the moisture sensor 120. In block 906, the disposable incontinence detection system 100 determines whether a circuit has been closed due to the presence of a biofluid bridging a space between the trace 132 and another trace that is coupled to the moisture sensor 120. If the circuit is not closed, the method 906 loops back to block 904 in which the disposable incontinence detection system 100 continues to apply the voltage to the trace 132. In the illustrative embodiment, when the circuit is closed, the moisture sensor 120 may detect a change in a voltage difference between the trace 132 and one of the other traces 134, 136, indicating that the circuit has been closed. In block 908, the incontinence detection system 100 generates a notification that an incontinence event has occurred. In doing so, the disposable incontinence detection system 100, and in particular, the notification device 122, generates a visual signal, as indicated in block 910, an audible signal, as indicated in block 912, and/or an electronic data communication, as indicated in block 914.

In some embodiments, the closure of the circuit provides the electrical power used by the notification device 122 to generate a notification. For example, the notification device 122, and in particular, the visual output device 308, may produce a visual signal (e.g., a light) using the electrical current passing through the trace 132 and the receiving trace(s) (e.g., one or more of the traces 134, 136). Accordingly, the conductivity of the biofluid, which may be a function of the type and/or volume of the biofluid, may affect the brightness or other characteristics of the visual signal produced by the visual output device 308. Accordingly, a caregiver may be able to determine the type and/or amount of biofluid based on the characteristics of the visual signal. In other embodiments, the moisture sensor 120 detects the closure of the circuit using the processor 302 such as by iteratively determining the voltage at each trace 130 and selectively activates one or more of the visual output device 308, the audio output device 310, or the data communication system 312 in accordance with a configuration stored in the main memory 304 and/or the data storage device 314. While the method 900 is described above with reference to the disposable incontinence detection system 100, it should be understood that other embodiments of the disposable incontinence detection system (e.g., the disposable incontinence detection systems 400, 600) consistent with the present disclosure may also perform the method 900.

Some of the above embodiments may be described in terms of functional block components and various processing steps. Such functional blocks may be realized by any number of hardware and/or software components configured to perform the specified functions. For example, embodiments may employ various integrated circuit components, e.g., memory elements, processing elements, logic elements, look-up tables, and the like, which may carry out a variety of functions under the control of one or more processors, microprocessors or other control devices. Similarly, where the elements of the above embodiments are implemented using software programming or software elements the embodiments may be implemented with any programming or scripting language such as C, C++, Java, assembler, or the like, with the various algorithms being implemented with any combination of data structures, objects, processes, routines or other programming elements. Furthermore, the embodiments could employ any number of conventional techniques for electronics configuration, signal processing and/or control, data processing and the like. The word "mechanism" may be used broadly and is not limited to mechanical or physical embodiments, but can include software routines in conjunction with processors, etc.

The particular implementations shown and described herein are illustrative examples and are not intended to otherwise limit the scope of the claims in any way. For the sake of brevity, conventional electronics, control systems, software development and other functional aspects of the systems (and components of the individual operating components of the systems) may not be described in detail. Furthermore, the connecting lines, or connectors shown in the various figures presented are intended to represent exemplary functional relationships and/or physical or logical couplings between the various elements. It should be noted that many alternative or additional functional relationships, physical connections or logical connections may be present in a practical device. Moreover, no item or component disclosed herein is intended to be an essential element. Numerous modifications and adaptations will be readily apparent to those skilled in this art.

The order of execution or performance of the operations in embodiments illustrated and described herein is not essential. That is, the operations may be performed in any order, unless otherwise specified, and embodiments as described may include additional or fewer operations than those disclosed herein. For example, executing or performing a particular operation before, contemporaneously with, or after another operation is contemplated.

Embodiments may be implemented with computer-executable instructions. The computer-executable instructions may be organized into one or more computer-executable components or modules. Embodiments may be implemented with any number and organization of such components or modules. For example, embodiments are not limited to the specific computer-executable instructions or the specific components or modules illustrated in the figures and/or described herein. Other embodiments may include different computer-executable instructions or components having more or less functionality than illustrated and described herein.

Although certain illustrative embodiments have been described in detail above, variations and modifications exist.

Embodiments of the invention can be described with reference to the following numbered clauses, with preferred features laid out in the dependent clauses:
1. A disposable incontinence detection system for monitoring an area for incontinence events, the incontinence detection system comprising:
   a substrate having a length and a width defining a monitoring area; and
   a moisture sensor positioned within the monitoring area and including a circuit that includes:
      a plurality of traces spaced apart from each other such that the presence of moisture bridging a space between at least two of the traces will close the circuit;
      a removable conductor coupled to one of the plurality of traces such that removal of the conductor irreversibly prevents the trace from closing the circuit.
   wherein the moisture sensor is configured to determine whether the circuit has been closed.
2. The disposable incontinence detection system of clause 1, wherein the removable conductor is one of a plurality of removable conductors and the plurality of traces comprise:
   a first outer trace;
   a second outer trace positioned opposite the first outer trace, and
   one or more inner traces positioned between the first outer trace and the second outer trace, wherein each of the inner traces includes a respective one of the plurality of removable conductors.
3. The disposable incontinence detection system of either clause 1 or clause 2, wherein the plurality of traces comprise:
   a first electrically conductive trace;
   a second electrically conductive trace spaced apart from the first electrically conductive trace by a predetermined distance; and
   a third electrically conductive trace positioned between the first electrically conductive trace and the second electrically conductive trace;
   wherein the removable electrical conductor is coupled to the third electrically conductive trace.
4. The disposable incontinence detection system of any preceding clause, wherein the plurality of traces includes at least four electrically conductive traces, wherein the removable conductor is a first removable conductor of a plurality of removable conductors and two of the electrically conductive traces each include a respective one of the removable conductors.
5. The disposable incontinence detection system of any preceding clause, wherein the plurality of traces are spaced apart at predefined distances associated with predefined volumes of liquid.
6. The disposable incontinence detection system of any preceding clause, wherein the removable conductor is coupled to the substrate by an adhesive.
7. The disposable incontinence detection system of any preceding clause, wherein each trace comprises a first segment and one or more second segments oriented perpendicular to the first segment.
8. The disposable incontinence detection system of clause 7, wherein the second segments of one of the plurality of traces are interdigitated with the second segments of another of the plurality of traces.
9. The disposable incontinence detection system of any preceding clause, wherein the moisture sensor is configured to apply a predefined voltage to one of the plurality of traces.
10. The disposable incontinence detection system of any preceding clause, wherein the sensor is configured to generate, in response to a determination that the circuit has been closed, a visual indication that an incontinence event has occurred.
11. The disposable incontinence detection system of any preceding clause, wherein the sensor is configured to generate, in response to a determination that the circuit has been closed, an audible indication that an incontinence event has occurred.
12. The disposable incontinence detection system of any preceding clause, wherein the sensor is further configured to transmit, in response to a determination that the circuit has been closed, a wireless signal that indicates that an incontinence event has occurred.
13. The disposable incontinence detection system of any preceding clause, wherein the traces comprise electrically conductive ink.
14. The disposable incontinence detection system of any preceding clause, wherein the substrate further comprises a pad having a plurality of layers including a top non-woven layer configured to be contacted by a user of the pad;
   wherein the moisture sensor is at least partially incorporated into one of the plurality of layers of the pad.
15. The disposable incontinence detection system of clause 14, wherein the plurality of layers further comprise an absorbent layer, a moisture sensor layer, a film layer, and a barrier layer.
16. The disposable incontinence detection system of any preceding clause, wherein the substrate is wearable.
17. A method for modifying the sensitivity of a disposable incontinence detection system that includes a substrate having a length and a width defining a monitoring area, and a moisture sensor that is positioned within the monitoring area and includes a circuit having a plurality of traces spaced apart from each other such that the presence of moisture bridging a space between at least two of the traces will close the circuit, a removable conductor coupled to one of the plurality of traces such that removal of the conductor irreversibly prevents the trace from closing the circuit, wherein the moisture sensor is configured to determine whether the circuit has been closed, the method comprising:
   irreversibly removing the conductor from the trace.
18. The method of clause 17, wherein the plurality of traces includes at least four electrically conductive traces, wherein the removable conductor is a first removable conductor of a plurality of removable conductors, and wherein two of the traces each includes a respective one of the removable conductors, the method further comprising:
   determining a threshold volume of liquid to be indicative of an incontinence event;
   selecting one or more of the conductors to remove based on the determined threshold volume of liquid; and
   wherein removing the conductor from the trace comprises removing the one or more selected conductors from the corresponding traces.
19. The method of either clause 17 or clause 18, further comprising positioning the substrate on a patient support apparatus.
20. The method of any one of clauses 17 to 19, further comprising:
   detecting a notification generated by the moisture sensor; and
   determining, in response to the detection of the notification, that an incontinence event has occurred.
21. The method of any one of clauses 17 to 20, wherein the conductor is coupled to the moisture sensor by an adhesive and removing the conductor from the trace comprises pulling the conductor from the moisture sensor.

## Claims

1. A disposable incontinence detection system for monitoring an area for incontinence events, the incontinence detection system comprising:
a substrate having a length and a width defining a monitoring area; and
a moisture sensor positioned within the monitoring area and including a circuit that includes:
a plurality of traces spaced apart from each other such that the presence of moisture bridging a space between at least two of the traces will close the circuit;
a removable conductor coupled to one of the plurality of traces such that removal of the conductor irreversibly prevents the trace from closing the circuit.
wherein the moisture sensor is configured to determine whether the circuit has been closed.

2. The disposable incontinence detection system of claim 1, wherein the removable conductor is one of a plurality of removable conductors and the plurality of traces comprise:
a first outer trace;
a second outer trace positioned opposite the first outer trace, and
one or more inner traces positioned between the first outer trace and the second outer trace, wherein each of the inner traces includes a respective one of the plurality of removable conductors.

3. The disposable incontinence detection system of either claim 1 or claim 2, wherein the plurality of traces comprise:
a first electrically conductive trace;
a second electrically conductive trace spaced apart from the first electrically conductive trace by a predetermined distance; and
a third electrically conductive trace positioned between the first electrically conductive trace and the second electrically conductive trace;
wherein the removable electrical conductor is coupled to the third electrically conductive trace.

4. The disposable incontinence detection system of any preceding claim, wherein the plurality of traces includes at least four electrically conductive traces, wherein the removable conductor is a first removable conductor of a plurality of removable conductors and two of the electrically conductive traces each include a respective one of the removable conductors.

5. The disposable incontinence detection system of any preceding claim, wherein the plurality of traces are spaced apart at predefined distances associated with predefined volumes of liquid.

6. The disposable incontinence detection system of any preceding claim, wherein the removable conductor is coupled to the substrate by an adhesive.

7. The disposable incontinence detection system of any preceding claim, wherein each trace comprises a first segment and one or more second segments oriented perpendicular to the first segment.

8. The disposable incontinence detection system of claim 7, wherein the second segments of one of the plurality of traces are interdigitated with the second segments of another of the plurality of traces.

9. The disposable incontinence detection system of any preceding claim, wherein the moisture sensor is configured to apply a predefined voltage to one of the plurality of traces.

10. The disposable incontinence detection system of any preceding claim, wherein the sensor is configured to generate, in response to a determination that the circuit has been closed, a visual indication that an incontinence event has occurred and/or an audible indication that an incontinence event has occurred.

11. The disposable incontinence detection system of any preceding claim, wherein the sensor is further configured to transmit, in response to a determination that the circuit has been closed, a wireless signal that indicates that an incontinence event has occurred.

12. The disposable incontinence detection system of any preceding claim, wherein the traces comprise electrically conductive ink.

13. The disposable incontinence detection system of any preceding claim, wherein the substrate further comprises a pad having a plurality of layers including a top non-woven layer configured to be contacted by a user of the pad;
wherein the moisture sensor is at least partially incorporated into one of the plurality of layers of the pad.

14. The disposable incontinence detection system of claim 13, wherein the plurality of layers further comprise an absorbent layer, a moisture sensor layer, a film layer, and a barrier layer.

15. The disposable incontinence detection system of any preceding claim, wherein the substrate is wearable.
